# EUROPÄISCHE PATENTSCHRIFT

(11) **EP 1 694 662 B1**
(45) Veröffentlichungstag und Bekanntmachung des Hinweises auf die Patenterteilung: **05.09.2012**
(21) Anmeldenummer: 04803523.2
(22) Anmeldetag: 04.12.2004
(51) Int. Cl.: C07D 307/08, C07D 307/32

(54) **Verfahren zur Herstellung von definierten Gemischen aus THF, BDO und GBL durch Gasphasenhydrierung**
Method for the production of defined mixtures of THF, BDO and GBL by gas phase hydrogenation
Procédé de preparation de melanges définis de THF, BDO, et GBL par hydrogénation en phase gazeuse

(30) Priorität: 09.12.2003 DE 10357715
(43) Veröffentlichungstag der Anmeldung: 30.08.2006
(73) Patentinhaber: BASF SE, 67056 Ludwigshafen (DE)
(72) Erfinder: RÖSCH, Markus, 55276 Dienheim (DE); PINKOS, Rolf, 67098 Bad Dürkheim (DE); HESSE, Michael, 67549 Worms (DE); SCHLITTER, Stephan, 67117 Limburgerhof (DE); JUNICKE, Henrik, 68165 Mannheim (DE); SCHUBERT, Olga, 67063 Ludwigshafen (DE); WECK, Alexander, 67251 Freinsheim (DE); WINDECKER, Gunther, 67067 Ludwigshafen (DE)
(86) Internationale Anmeldenummer: PCT/EP2004/013811
(87) Internationale Veröffentlichungsnummer: WO 2005/058853

(56) Entgegenhaltungen:
- WO-A-97/43234
- WO-A-99/35136
- DE-A1- 10 061 556

## Beschreibung

Die vorliegende Erfindung betrifft ein Verfahren zur Herstellung von gegebenenfalls alkylsubstituiertem Gemischen aus Tetrahydrofuran (THF), Butandiol (BDO) und/oder Butyrolacton (GBL) durch katalytische Hydrierung in der Gasphase von Substraten, die ausgewählt sind aus der Gruppe bestehend aus Derivaten der Maleinsäure und Bernsteinsäure sowie diesen Säuren selbst. Unter Derivaten werden im Rahmen der vorliegenden Erfindung Anhydride verstanden, die ebenso wie die Säuren einen oder mehrere Alkylsubstituenten aufweisen können.

Die Hydrierung von MSA führt über die Zwischenstufe Bersteinsäureanhydrid (BSA) zu Butyrolacton (GBL) und anschließend zu THF. Überhydrierungsreaktion führen zu n-Butanol (BuOH) und n-Butan. GBL steht mit BDO in einem druck- und temperaturabhängigen Gleichgewicht, welches bei niedrigen Temperaturen und hohen Drucken auf Seiten des Butandiols liegt. Das gewünschte Produkt Butandiol kann durch Cyclisierung zu THF und durch Überhydrierung zu Butanol und Butan abreagieren.

Die Gasphasenhydrierung von gereinigtem Maleinsäureanhydrid (MSA) zu Butyrolacton (GBL) und/oder THF und die Umsetzung von gereinigtem GBL zu BDO sind zwei seit vielen Jahren bekannte Reaktionen. Zur Durchführung jeder dieser katalytischen Reaktionen sind in der Literatur zahlreiche Katalysatorsysteme beschrieben. Je nach Wahl der Katalysatoren und der gewählten Reaktionsparameter werden mit derartigen Katalysatoren unterschiedliche Produktgemische erreicht. Verfahren zur Herstellung von Butandiol ausgehend von MSA sind ebenfalls bereits bekannt.

Sollen GBL und BDO hergestellt werden, die Alkylsubstituenten aufweisen, so bietet es sich an, von den vorstehend genannten Edukten auch die entsprechenden alkylsubstituierten Spezies zu verwenden.

Für die verschiedenen Stufen der Hydrierung von MSA zu den Produkten THF, GBL und BDO sind vielfältige Katalysatoren bekannt, die insbesondere bei älteren Verfahren, häufig chromhaltig sind. Die Hydrierung ist in den meisten Fällen auf MSA als Edukt beschränkt.

Aus US 5149836 ist ein Mehrstufen-Gasphasenverfahren zur Herstellung von GBL und THF mit innerhalb der Grenzen: 15 % - 92 % GBL und 7 % - 83 % THF: variablen Produktselektivitäten bekannt. Dabei wird in einer ersten Stufe eine Mischung aus Rein-MSA und Wasserstoff über einen Katalysator geleitet wird welcher Kupfer, Zink und Aluminium enthält. Dieser rohe Reaktionsaustrag wird anschließend über einen chromhaltigen Katalysator geführt. Das Verhältnis GBL zu THF wird durch die Variation der der Kontaktzeit im zweiten Reaktor, der Temperatur und der Katalysatormengen zueinander beeinflusst.

WO 99/35136 offenbart ein zweistufiges Verfahren zur Herstellung von GBL und THF, bei dem MSA in einer ersten Stufe mit einem Kupfer-haltigen Katalysator hydriert wird. Der so erhaltene Reaktionsaustrag wird über einen sauren Silizium-Aluminium-haltigen Katalysator geleitet. Der Anteil an GBL und BDO beträgt 60 %. Nachteilig an diesem Verfahren ist der starre Produktmix.

Ein Verfahren zur gleichzeitigen Herstellung von BDO und THF ausgehend von MSA an Kupfer-, Chrom- und Mangan-enthaltenden Katalysatoren ist in EP -A 0 373 947 offenbart. Bevorzugt werden gemäß den Ausführungsbeispielen Gemische aus MSA und GBL, Gemische aus MSA und 1,4-Dioxan und Rein-MSA eingesetzt. In allen Fällen werden THF-Ausbeuten von ca. 10 - 15 mol% erhalten. Nachteilig ist jedoch, dass ausschließlich Gemische aus BDO und THF, jedoch keine GBL-haltigen Gemische erhältlich sind. GBL wird lediglich bei einem geringen Wasserstoff-Edukt Überschuss und bei Drucken nahe Atmosphärendruck erhalten. Weiterhin sind die Katalysatorbelastungen so gering (0,03 - 0,04 kg_{MSA}/I_{Kat}h), dass das Verfahren unwirtschaftlich erscheint.

Die Verwendung eines chromfreier Katalysatoren auf der Basis gemischter Cu-Al-oxide wird in der JP-A-2 233 631 offenbart. Das Ziel dieser Erfindung liegt darin, die Hydrierung von MSA so durchzuführen, dass als Hauptprodukte THF und BDO neben nur geringen oder gar keinen Mengen von GBL entstehen. Dieses wird dann durch die Verwendung der auf gemischten Cu-Al-Oxiden basierenden Katalysatoren sowie durch Einhalten bestimmter Reaktionsbedingungen erreicht. Generelle Mengenangaben bezüglich des Cu-und Al-Oxides finden sich nicht; in den Beispielen sind 2 Katalysatorzusammensetzungen offenbart, eine mit ca. 46 Gew.-% CuO und 33 Gew.-% Al₂O₃, die andere mit ca. 36 Gew.-% CuO und 47 Gew.-% Al₂O₃. Gemäß den Beispielen wird die Hydrierung bei Temperaturen zwischen ca. 210 bis 230°C und GHSV-Werten von ca. 3200 bis 9600 durchgeführt. Die Wasserstoff/MSA-Verhältnisse liegen bei für industrielle Verfahren vergleichsweise ungünstig hohen Werten, in den Beispielen von 200 bis 800.

Die Hydrierung von MSA unter Bedingungen, die denen in der JP-A-2 233 631 entsprechen, jedoch unter Verwendung eines anderen Katalysators, werden in der JP-B2-2 639 463 offenbart. Die Verwendung des Katalysators soll die Herstellung von BDO und THF durch Hydrierung von MSA ermöglichen. Eingesetzt wird hierbei ein Kupferoxid/Zinkoxid/Aluminiumoxid-Katalysator, dessen Zusammensetzung in der Beschreibung nicht quantitativ dargelegt wird. Die nach den Beispielen verwendeten Katalysatoren weisen eine Zusammensetzung von 20 Gew.-% CuO, 43,6 Gew.-% ZnO und 18,1 Gew.-% Al₂O₃, 32,6 Gew.-% CuO 38,1 Gew.-% ZnO und 9,5 Gew.-% Al₂O₃, 24,2 Gew.-% CuO, 36,4 Gew.-% ZnO und 17,2 Gew.-% Al₂O₃, 26,4 Gew.-% CuO, 52,9 Gew.-%, ZnO, 7,6 Gew.-% Al₂O₃ und 1,4 Gew.-% CaO sowie 22,9 Gew.-% CuO, 44,8 Gew.-% ZnO und 16,3 Gew.-% Al₂O₃ auf. Es wird generell in einem Lösungsmittel wie GBL oder Dioxan gearbeitet.

Die den oben zitierten Druckschriften zugrundeliegenden Technologien benutzen als Edukt für die Hydrierungsreaktionen vorgereinigtes MSA, das nach seiner Herstellung im allgemeinen durch Destillation von Verunreinigungen befreit wurde. MSA wird hergestellt durch partielle Oxidation von bestimmten Kohlenwasserstoffen, nämlich Benzol, Butengemischen sowie n-Butan, wobei dieses letztere vorzugsweise eingesetzt wird. Das Rohprodukt der Oxidation enthält neben dem gewünschten MSA vor allem Nebenprodukte wie Wasser, Kohlenmonoxid, Kohlendioxid, nicht umgesetzten Ausgangskohlenwasserstoff sowie Essig-und Acrylsäure, wobei diese Nebenprodukte unabhängig von den in die Oxidation eingesetzten Kohlenwasserstoffen sind. Zumeist werden die Nebenprodukte durch aufwendige Verfahren abgetrennt, beispielsweise durch Destillation, wie oben erwähnt. Das Reinigen erweist sich insbesondere als notwendig, weil die in den Hydrierverfahren eingesetzten Katalysatoren generell empfindlich auf solche Verunreinigungen reagieren. Die Desaktivierung der Katalysatoren ist bereits bei Einsatz von gereinigtem MSA ein Problem, da durch Belegung mit dessen Polymerisationsprodukten der Katalysator in der Regel in relativ kurzen Intervallen, die oftmals bei ca. 100 Stunden liegen, regeneriert werden muss. Die Tendenz zur Desaktivierung ist beim Vorliegen polymerisierbarer Verunreinigungen, wie beispielsweise der Acrylsäure, noch erhöht. Diese Tatsache ist dem Fachmann bekannt und wird beispielsweise auch in der Patentanmeldungen EP-A 322140 beschrieben.

Weiterhin existieren im Stand der Technik wenig Druckschriften, die die Hydrierung von lediglich grob vorgereinigtem MSA offenbaren.

Aus der DE 10061556 und der WO 97/43234 ist bekannt, Maleinsäureanhydrid aus Maleinsäureanhydrid enthaltenden Gasströmen, die aus der Oxidation von Kohlenwasserstoffen stammen, mit Hilfe von mindestens 30°C höher siedenden Absorptionsmitteln zu absorbieren, das Maleinsäureanhydrid aus diesen Absorptionsmitteln mit Hilfe von Wasserstoff herauszutreiben und den Maleinsäureanhydrid enthaltenden Wasserstoffstrom in der Gasphase an einem heterogenen Katalysator zu hydrieren. Bei WO 97/43234 wird hauptsächlich BDO neben geringen Mengen GBL und THF erhalten. Die Hydrierung wird bei etwa 150°C bis 300°C und einem Druck von 5 bar bis 100 bar in der Gasphase durchgeführt. Als Katalysatoren werden promotierte Kupferkatalysatoren verwendet, wie sie in Journal of Catalysis 150, Seiten 177 bis 185 (1994) beschrieben sind. Dabei handelt es sich um chromhaltige Katalysatoren des Typs Cu/MnBa/Cr und Cu/Zn/Mg/Cr. Somit werden gemäß der Offenbarung dieser Anmeldung chromhaltige Katalysatoren zur Hydrierung von MSA-Qualitäten, die die oben dargelegten Verunreinigungen aufweisen, eingesetzt. Der Einsatz chromhaltiger Katalysatoren wird jedoch aufgrund der Toxizität heutzutage so weit wie möglich vermieden. Bei DE 10061556 wird in hohen Ausbeuten THF erhalten. GBL tritt als Nebenkomponenente auf, BDO wird nicht beobachtet.

Die Aufgabe der vorliegenden Erfindung liegt darin, ein Verfahren bereitzustellen, mit dem gezielt Gemische aus BDO, GBL und/oder THF mit definierter Zusammensetzung, das heißt mit vom Betreiber des Verfahrens nach Wunsch festgelegtem Anteil der drei Produkte BDO; GBL und THF, ausgehend von MSA hergestellt werden können, wobei je nach Produktwunsch ohne Veränderung der Anlage oder des Katalysators unterschiedliche Mengen BDO, GBL und THF erhalten werden können. Dieses Verfahren soll dabei kontinuierlich durchführbar sein und möglichst große Mengen der Wertprodukte BDO, GBL und THF liefern, um so eine größtmögliche Wirtschaftlichkeit zu erreichen. Weiterhin sollte der Katalysator mit MSA betrieben werden können, das nicht aufwendig vorgereinigt wurde, beispielsweise durch Destillation, und trotzdem eine hohe Standfestigkeit aufweisen, also kein häufiges Regenerieren erfordern. Das Verfahren soll dem Betreiber die Variabilität ermöglichen ohne einen Katalysatorwechsel, d.h. nur durch Änderung der Betriebsparameter, ein verändertes Produktgemisch zu erhalten, um sich flexibel und ökonomisch an die Markterfordernisse anpassen zu können. Alle zugänglichen Produktgemische sollen möglichst BSA-frei sein, um einerseits verfahrenstechnische Probleme (Verlegungen in Rohrleitungen) zu verhindern, andererseits aber auch Ausbeuteverluste zu minimieren. Der eingesetzte Katalysator sollte kostengünstig und edelmetallfrei sein. Die Belastbarkeit des Katalysators soll möglichst hoch sein. Das MSA soll ohne Lösemittel in die Hydrierung eingesetzt werden, um eine Rückführung des Lösemittels zu vermeiden. Des weiteren sollten Flüssigphasenhydrierungen wässriger Maleinsäurelösungen aufgrund des unwirtschaftlich hohen Drucks und der Korrosivität des Feedstroms vermieden werden. Außerdem sollten Gasphasenverfahren bei Normaldruck vermieden werden, da bei solchen Verfahren unwirtschaftlich hohe Gasvolumina im Kreis bewegt werden müssen. Auch sollten mehrstufige Hydrierungen bei einer Druckstufe verwirklicht werden, um eine höhere Wirtschaftlichkeit zu erreichen.

Diese Aufgabe wird gelöst durch ein Verfahren zur variablen Herstellung von Gemischen von gegebenenfalls alkylsubstituiertem BDO, GBL und THF durch zweistufige Hydrierung in der Gasphase von C₄-Dicarbonsäuren und/oder deren Derivaten, dadurch gekennzeichnet, dass man
a) in einem ersten Schritt in der Gasphase einen Gasstrom von C₄-Dicarbonsäuren und/oder deren Derivaten an einem Katalysator bei einem Druck von 2 bis 100 bar und einer Temperatur von 200°C bis 300°C in einem ersten Reaktor in Gegenwart eines Katalysator in Form von Katalysatorformkörpern mit einem Volumen kleiner als 20 mm³, die 5 bis 95 Gew.-% Cu-Oxid und 5 bis 95 Gew.-% ei nes Oxids mit sauren Zentren zu einem hauptsächlich aus gegebenenfalls alkylsubstituiertem GBL und THF haltigen Strom hydriert,
b) eventuell entstandenes Bernsteinsäureanhydrid durch Partialkondensation abtrennt,
c) die bei der Partialkondensation überwiegend in der Gasphase verbliebenen Produkte THF, Wasser und GBL unter gleichem oder um die Strömungsverluste im Hydrierkreislauf verringerten Druck und einer Temperatur von 150 bis 240°C in einem zweiten Reaktor an einem Katalysator der 5 bis 95 Gew.-% CuO und 5 bis 95 Gew.-% eines oder mehrerer Oxide ausgewählt aus der Gruppe ZnO, Al₂O₃, SiO₂, TiO₂, ZrO₂, CeO₂, MgO, CaO, SrO, BaO und Mn₂O₃ zu einem Gemisch aus BDO, GBL und THF enthaltenden Strom umsetzt.
d) den Wasserstoff von den Produkten abtrennt und in die Hydrierung zurückführt,
e) die Produkte THF, BDO, GBL und Wasser destillativ trennt, einen GBL-reichen Strom in den zweiten Reaktor gegebenenfalls zurückführt oder gegebenenfalls ausschleust und BDO, THF und GBL destillativ aufarbeitet.,
und das Verhältnis der Produkte THF, GBL und BDO zueinander im Bereich von 10 bis 100 Gew.-% THF, 0 bis 90 Gew.-% GBL und 0 bis 90 Gew.-% BDO nur durch Variation der Temperaturen in beiden Hydrierzonen sowie gegebenenfalls des GBL-Rückführstroms eingestellt wird.

Unter dem Begriff C₄-Dicarbonsäuren und deren Derivate werden im Bezug auf die vorliegende Anmeldung Maleinsäure und Bernsteinsäure, die gegebenenfalls einen oder mehrere C₁-C₆-Alkylsubstituenten aufweisen sowie die Anhydride dieser gegebenenfalls alkylsubstituierten Säuren verstanden. Ein Beispiel einer solchen Säure ist Citraconsäure. Vorzugsweise werden die jeweiligen Anhydride einer gegebenen Säure eingesetzt. Insbesondere ist das verwendete Edukt MSA.

Die dem Fachmann an sich vertraute Partialkondensation des BSA kann auch als Umlaufquenchkreis ausgeführt werden. Hierbei werden die Reaktionsgase mit dem Kondensat der Partialkondensation selbst gequencht.

Die Verdampfung des GBL-Rückführstroms aus Stufe e), das heißt das rückgeführte GBL oder GBL/Wasser-Gemisch kann in einem Gegenstromkontaktapparat, vorzugsweise einer Strippkolonne, mit dem GBL/THF-beladenen Kreisgaswasserstoff erfolgen.

Die Partialkondensation des Bersteinsäureanhydrides und die Verdampfung des GBL oder GBL/Wasser-Rückführstromes kann auch in einem Apparat kombiniert werden, bevorzugt in einer Gegenstrom-Strippkolonne mit externem Quenchkreislauf, wobei das Bernsteinsäureanhydrid zusammen mit Rest GBL, Wasser und hochsiedenden Nebenkomponenten als Sumpfaustrag ausgeschleust werden kann.

Am besten eignet sich dieses Verfahren zum kontinuierlichen Betrieb.

Der aus dem zweiten Reaktor austretende Gasstrom wird erfindungsgemäß auf 10 bis 60°C gekühlt. Dabei werden die Reaktionsprodukte auskondensiert und in einen Abscheider geleitet. Der nicht-kondensierte Gasstrom wird vom Abscheider abgezogen und dem Kreisgasverdichter zugeführt. Eine kleine Kreisgasmenge wird ausgeschleust. Die auskondensierten Reaktionsprodukte werden dem System kontinuierlich entnommen und einer Aufarbeitung zugeführt. Als Nebenprodukte findet man in der auskondensierten Flüssigkeitsphase hauptsächlich n-Butanol neben geringen Mengen an Propanol.

Zum Erzielen der erfindungsgemäßen BDO, GBL und THF-Selektivitäten ist weiterhin das Einhalten gewisser Reaktionsparameter erforderlich.

Ein wichtiger Parameter ist das Einhalten einer geeigneten Reaktionstemperatur im ersten Reaktor. Dies wird zum einen erreicht durch eine genügend hohe Eingangstemperatur der Edukte in den Reaktor. Diese liegt bei Werten von > 220 bis 300°C, vorzugsweise 235 bis 270°C. Um eine akzeptable bzw. hohe Wertprodukt-Selektivität und -Ausbeute zu erhalten, muss die Reaktion so durchgeführt werden, dass am Katalysatorbett, an dem die eigentliche Reaktion stattfindet, eine geeignet hohe Reaktionstemperatur herrscht. Über die Länge des Reaktors lässt sich bei gegebenem Druck, Wasserstoff/Edukt-Verhältnis und Belastung durch die Eingangstemperatur ein Temperaturprofil einstellen, wobei im ersten Reaktor mehrere Temperaturmaxima erreicht werden können. Diese sogenannten Hot-Spot-Temperaturen liegen bei Werten von 240 bis 310°C, vorzugsweise 240 bis 280°C. Das Verfahren wird so durchgeführt, dass die Eingangstemperatur der Reaktionsgase im ersten Reaktor unterhalb dieser Hot-Spot-Temperatur liegt. Der erste Hot-Spot liegt räumlich in der ersten Hälfte des ersten Reaktors nach dem Eintrittspunkt des Reaktionsgemischs, insbesondere bei Vorliegen eines Rohrbündelreaktors. Vorzugsweise liegt die erste Hot-Spot-Temperatur 5 bis 15°C, insbesondere 10 bis 15°C, oberhalb der Eintrittstemperatur. Falls ein zweiter Hot-Spot im ersten Reaktor auftritt, kann sich dieser an jeder Stelle des Reaktors befinden und wird durch ein Absenken der Eintrittstemperatur räumlich hin zum Reaktorausgang verschoben. Vorzugsweise liegt die zweite Hot-Spot-Temperatur 1 bis 15°C, insbesondere 2 bis 10°C, oberhalb der Eintrittstemperatur. Sollen hauptsächlich THFhaltige Gemische erhalten werden, befindet sich dieser zweite Hot-spot bevorzugt in den ersten 2/3 des ersten Reaktors, sollen bevorzugt GBL- und BDO-haltige Gemische erhalten werden, befindet sich der zweite Hotspot im letzten Drittel, insbesondere am Reaktorausgang des ersten Reaktors.

Wird die Hydrierung unterhalb der Minimaltemperaturen der Eingangs- bzw. Hot-Spot-Temperatur durchgeführt, ist im Verlauf der Hydrierung eine Desaktivierung des Katalysators durch Belegung mit Bernsteinsäure, Fumarsäure und/oder BSA zu beobachten. Wird dagegen mit MSA als Edukt oberhalb der Maximaltemperaturen der Eingangs- bzw. Hot-Spot-Temperatur hydriert, sinken die GBL und THF-Ausbeute und Selektivität auf nicht zufriedenstellende Werte. Es ist hierbei die vermehrte Bildung von n-Butanol und n-Butan zu beobachten, also den Produkten einer weiteren Hydrierung.

Ein weiterer wichtiger Parameter ist das Einhalten einer geeigneten Eingangstemperatur im zweiten Reaktor. Diese liegt bei Werten zwischen 150°C und 270°C, vorzugsweise 175°C bis 195°C. Wenn die Hydrierung unterhalb der Minimaltemperaturen der Eingangstemperatur durchgeführt wird, dann steigt die Menge an GBL an. Der Katalysator verliert an Aktivität. Weiterhin ist unterhalb der Minimaltemperatur mit einem Auskondensieren der Einsatzstoffe und einer nachhaltigen Schädigung des Kupferkatalysators durch Wasser zu rechnen. Wird oberhalb der Maximaltemperaturen der Eingangstemperatur hydriert, sinken die BDO-Ausbeute und Selektivität auf nicht zufriedenstellende Werte. Die Nebenproduktbildung durch Überhydrierung zu n-Butanol und n-Butan ist jedoch bei höherer Temperatur verstärkt zu beobachten.

Die Temperaturerhöhung des Gasstromes im zweiten Reaktor darf 90°C nicht überschreiten, vorzugsweise darf die Temperaturerhöhung nicht 40°C überschreiten, insbesondere nicht mehr als 20°C. Auch hier führen zu hohe Temperaturerhöhungen zu Überhydrierreaktionen und einem (BDO, THF + GBL) Selektivitätsverlust.

Der Druckbereich ist für die BDO-Ausbeute von entscheidender Wichtigkeit. Im Zusammenspiel mit der Temperatur und der Wasserstoffmenge ist darauf zu achten, dass alle Einsatzstoffe in der Gasphase vorliegen.

In der Hydrierung wird ein Druck von 2 bis 100 bar, bevorzugt ein Druck von 2 bis 60 und besonders bevorzugt ein Druck von 15 bis 35 bar gewählt. In diesem Druckbereich verschiebt sich im zweiten Reaktor mit steigendem Druck das Verhältnis GBL zu BDO hin zu BDO. Wird unterhalb des Druckbereichs gearbeitet wird hauptsächlich GBL aus dem zweiten Reaktor getragen und somit der Rückführstrom unwirtschaftlich groß.

Die Katalysatorbelastung der erfindungsgemäßen Hydrierungsstufen liegt jeweils im Bereich von 0,02 bis 2 kg Edukt/I Katalysator · Stunde. Vorzugweise liegt die Katalysatorbelastung im Bereich von 0,05 bis 1 kg Edukt/I Katalysator · Stunde. Wird die Katalysatorbelastung im Falle von MSA als Feed über den genannten Bereich hinaus erhöht, ist generell eine Erhöhung des Anteils an BSA und Bernsteinsäure im Hydrieraustrag des ersten Reaktors zu beobachten. Wird die Belastung unter die Minimalbelastung gesenkt, treten verstärkt Überhydrierprodukte auf, des weiteren wird das Verfahren aufgrund der niedrigen Belastungen unwirtschaftlich.

Das Wasserstoff/Edukt-Molverhältnis ist ebenfalls ein Parameter, der einen wichtigen Einfluss auf die Produktverteilung und auch die Wirtschaftlichkeit des erfindungsgemäßen Verfahrens hat. Aus wirtschaftlicher Sicht ist ein niedriges Wasserstoff/Edukt-Verhältnis wünschenswert. Die Untergrenze liegt bei einem Wert von 5, wobei jedoch generell höhere Wasserstoff/Edukt-Molverhältnisse von 20 bis 650 angewendet werden. Der Einsatz der oben beschriebenen, erfindungsgemäßen Katalysatoren sowie das Einhalten der oben beschriebenen Temperaturwerte erlaubt den Einsatz günstiger, niedriger Wasserstoff/Edukt-Verhältnisse bei der Hydrierung der ersten Stufe, die vorzugsweise bei Werten von 20 bis 200, vorzugsweise 40 bis 150 liegen. Der günstigste Bereich liegt bei Werten von 50 bis 100.

Im zweiten Hydrierreaktor wird GBL zu BDO umgesetzt. Hier wird das Molverhältnis Wasserstoff/GBL in der zweiten Hydrierstufe bei Werten von 20 bis 1000, vorzugsweise 50 bis 400, insbesondere 100 bis 300. gewählt. Durch diese Molverhältnisse ist der Austrag der entstehenden Reaktionswärme aus dem Reaktor der zweiten Stufe durch das Kreisgas und ohne externe Wärmeabfuhr möglich.

Um die erfindungsgemäß verwendeten Wasserstoff/Edukt-Molverhältnisse einzustellen, wird ein Teil, vorteilhafterweise die Hauptmenge, des Wasserstoffs im Kreis gefahren. Hierzu setzt man im allgemeinen die dem Fachmann bekannten Kreisgasverdichter ein. Die chemisch durch die Hydrierung verbrauchte Wasserstoffmenge wird ergänzt. In einer bevorzugten Ausführungsform wird ein Teil des Kreisgases ausgeschleust, um Inertverbindungen, beispielsweise n-Butan, zu entfernen. Der im Kreis geführte Wasserstoff kann auch, gegebenenfalls nach Vorheizen, zum Verdampfen des Eduktstroms benutzt werden.

Gemeinsam mit dem Wasserstoff-Kreisgas werden alle Produkte im Kreis geführt, die beim Kühlen des aus dem Hydrierreaktor austretenden Gasstroms nicht oder nicht vollständig auskondensieren. Dies sind vor allem THF, Wasser und Nebenprodukte wie Methan und Butan. Die Kühltemperatur beträgt 0 bis 60°C, vorzugsweise 20 bis 45°C.

Es können mehrere Reaktoren parallel oder hintereinander geschaltet eingesetzt werden. Als Reaktortypen kommen alle, für heterogen katalysierte Reaktionen mit einem gasförmigen Edukt- und Produktstrom geeigneten Apparate in Betracht. Bevorzugt sind Rohrreaktoren, Schachtreaktoren oder Reaktoren mit innerer Wärmeabfuhr, beispielsweise Rohrbündelreaktoren. Besonders bevorzugt eingesetzt werden in der ersten Hydrierstufe Rohrbündelreaktoren und in der zweiten Hydrierstufe Schachtreaktoren eingesetzt.

Prinzipiell kann zwischen die Katalysatorbetten eine Zwischeneinspeisung erfolgen. Möglich ist auch eine Zwischenkühlung zwischen oder in den Katalysatorbetten bzw. Reaktoren. Bei Einsatz von zwei Reaktoren mit unterschiedlichem Temperaturbereich kann die GBL- Rückführung zwischen beiden Reaktoren erfolgen. Bei Einsatz von Festbettreaktoren ist eine Verdünnung des Katalysators durch Inertmaterial möglich.

Für das erfindungsgemäße Verfahren werden in beiden Hydrierstufen verschiedene Katalysatoren verwendet.

Der erfindungsgemäße Katalysator der ersten Hydrierstufe zeichnet sich dadurch aus, dass er in der Lage ist hinsichtlich ihrer Produktverhältnisse variable Gemische aus GBL und THF durch Gasphasenhydrierung von MSA zugänglich zu machen, die weitgehend BSA frei sind. Er besitzt eine hohe Standzeit und eine hohe Belastbarkeit, so dass Reaktivierungen unterlassen und Reaktorvolumen eingespart werden kann. Alle diese Punkte machen ein mit diesem Katalysator ausgeübtes Verfahren besonders wirtschaftlich.

Der erfindungsgemäße Katalysator der ersten Hydrierstufe weist als Hauptbestandteil Kupferoxid und ein saures oxidisches Material auf und kann optional eines oder mehrere weitere Metalle bzw. deren Verbindungen, vorzugsweise Oxide, aus den Gruppen 1 bis 14 (IA bis VIIIA und IB bis IVB der alten IUPAC-Nomenclatur) des Periodensystems der Elemente enthalten. Wird ein solches weiteres Oxid verwendet, wird vorzugsweise TiO₂, ZrO₂, SiO₂, CaO, Na₂O, Mn₂O₃, BaO, und/oder MgO eingesetzt.

Der Anteil des Kupferoxids an der Gesamtmasse des Katalysators liegt bei 5 bis 95 Gew.%, vorzugsweise bei < 70 Gew.%., besonders bevorzugt 10 bis 65 Gew.-%.

Der erfindungsgemäße Katalysator der ersten Hydrierstufe enthält ein saures Oxid, das eine geeignete Anzahl an sauren Zentren aufweisen muss. Dabei ist der erforderliche Mengeanteil an dem Oxid mit sauren Zentren von der darin enthaltenen Menge an sauren Zentren abhängig. Geeignete saure Oxide mit einer ausreichenden Zahl an sauren Zentren sind Titandioxid, Zirkoniumdioxid, Siliciumdioxid und Aluminiumoxid, dessen Verwendung bevorzugt ist und Mischoxide aus diesen Oxiden. Besonders bevorzugte Katalysatorzusammensetzungen weisen < 70 Gew.-% Kupferoxid, insbesondere 10 bis 65 Gew.-% CuO, und > 20 Gew.%, bevorzugt > 30 Gew.-%, insbesondere 35 bis 90 Gew.-%, saures Oxid, auf. Niedrige Kupferoxid-Gehalte sind auch aufgrund des damit erzielten Kostenvorteils bevorzugt. Durch die sauren Oxide lassen sich hohe Ausbeuten erzielen. Die Metalle aus den Gruppen 1 bis 14 des Periodensystems der Elemente können in einer Menge von bis zu 30 Gew.-% im Katalysator enthalten sein.

Die eingesetzten Katalysatoren der ersten Hydrierstufe können zudem ein oder mehrere Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel, Zelluloseverbindungen, Stärke, Polyolefine, Kohlenhydrate (Zucker), Wachse, Alginate sowie Aluminiumoxid, Aluminiumoxidhydroxid, z.B. in Form von Böhmit; Zirkonoxid, Siliziumoxid und deren Sole, substituierte und nicht substituierte Siloxane und Kupferpulver.

Die Katalysatoren der ersten Hydrierstufe lassen sich nach dem Fachmann bekannten Methoden herstellen. Bevorzugt sind Verfahren, bei denen das Kupferoxid fein verteilt und innig vermischt mit den anderen Bestandteilen der Aktivmasse und dem sauren Oxid anfällt. Besonders bevorzugt werden die entsprechenden Metallsalze und/oder Hydroxide aus wässriger Lösung ausgefällt, gewaschen, getrocknet und calciniert. Als Metallsalze kommen beispielsweise Nitrate, Sulfate, Carbonate, Chloride, Acetate oder Oxalate in Betracht. Anschließend wird dieses Ausgangsmaterial nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Es ist auch möglich die erfindungsgemäßen Katalysatoren der ersten Hydrierstufe durch Aufbringen der Aktivmasse oder von Vorläuferverbindungen der Aktivmasse auf ein saures Oxid hergestellt werden, beispielsweise durch Tränken oder Aufdampfen. Weiterhin können erfindungsgemäße Katalysatoren durch Verformen einer Mischung aus Aktivkomponenten und saurem Oxid oder deren Vorläuferverbindung mit einem Oxid mit sauren Zentren oder einer Vorläuferverbindung hiervon erhalten werden.

Die Katalysatoren der ersten Hydrierstufe werden als Formkörper verwendet. Beispiele umfassen Stränge, Rippstränge, andere Extrudatformen, Tabletten, Ringe, Kugeln und Splitt.

Unter allen Formkörpern eignen sich für den Katalysator der ersten Hydrierstufe besonders Extrudate. Diese werden durch Extrudieren der calcinierten Ausgangsverbindung mit einem Hilfsmittel (Binder), beispielsweise Böhmit oder p-Böhmit (AIOOH) erhalten und anschließend calciniert. Der Binder kann vor dem Extrudieren vorbehandelt werden. Bevorzugt geschieht dies mit Säure, wie zum Beispiel mit Ameisensäure, Salpetersäure oder Salzsäure. Andere Hilfsmittel wie zum Beispiel Porenbildner wie Carboxymethylzellulose, Kartoffelstärke oder Stearinsäure können zusätzlich vor dem Extrudieren zugegeben werden.

Die Bestimmung des erfindungsgemäßen Volumens der Formkörper der ersten Hydrierstufe erfolgt dabei durch Berechnung aus den Abmessungen der Formkörper.

Für die zweite Hydrierstufe wird ein Katalysator genutzt, der als katalytisch aktiven Hauptbestandteil Kupferoxid aufweist und nur eine geringe Anzahl an sauren Zentren besitzen darf. Wird ein Katalysator mit einer zu hohen Anzahl an sauren Zentren verwendet, wird BDO dehydratisiert und es entsteht THF.

Ein geeignetes Oxidmaterial, das eine ausreichend geringe Anzahl an sauren Zentren besitzt, ist ein Material ausgewählt aus der Gruppe ZnO, Al₂O₃, SiO₂, TiO₂, ZrO₂, CeO₂, MgO, CaO, SrO, BaO, La₂O₃ und Mn₂O₃ und Mischungen davon. Bevorzugt sind als Trägermaterialien ZnO-/Al₂O₃-Gemische, die delta-, theta-, alpha- und eta-Modifikationen des Al₂O₃ sowie Mischungen, die Al₂O₃ und mindestens eine Komponente aus der Gruppe SiO₂, TiO₂, ZrO₂ einerseits und aus der Gruppe ZnO, MgO, CaO, SrO und BaO andererseits enthalten, enthalten. Besonders bevorzugt als Trägermaterialien sind reines ZnO, ZnO/Al₂O₃-Gemische im Gewichtsverhältnis 100:1 bis 1:2, sowie Mischungen von SiO₂ mit MgO, CaO und/oder ZnO im Gewichtsverhältnis 200:1 bis 1:1.

Die Menge an Kupferoxid liegt bei Werten ≤ 95 Gew.-%, vorzugsweise 5 bis 95 Gew.-%, insbesondere bei 15 bis 80 Gew.-%; das Oxidmaterial wird in Mengen von ≥ 5 Gew.-%, vorzugsweise 5 bis 95 Gew.-%, insbesondere 20 bis 85 Gew.-% eingesetzt.

Aufgrund der Toxizität chromhaltiger Katalysatoren werden in beiden Hydrierstufen bevorzugt chromfreie Katalysatoren eingesetzt. Selbstverständlich eignen sich technisch auch entsprechende, dem Fachmann bekannte chromhaltige Katalysatoren für einen Einsatz in dem erfindungsgemäßen Verfahren, wodurch sich jedoch nicht die gewünschten Vorteile, die insbesondere umwelt- und arbeitstechnischer Natur sind, ergeben.

Die eingesetzten Katalysatoren für die zweite Hydrierstufe können zudem ein Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel und Kupferpulver.

Die Katalysatoren der zweiten Hydrierstufe lassen sich nach dem Fachmann bekannten Methoden herstellen. Bevorzugt sind Verfahren, bei denen das Kupferoxid fein verteilt und innig vermischt mit den anderen Bestandteilen anfällt, besonders bevorzugt sind Auftränken und Fällungsreaktionen.

Diese Ausgangsmaterialien können nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Alternativ können erfindungsgemäße Katalysatoren der zweiten Hydrierstufe beispielsweise auch durch Aufbringen der Aktivkomponente auf einen Träger hergestellt werden, beispielsweise durch Beschichten oder Aufdampfen. Weiterhin können erfindungsgemäße Katalysatoren durch Verformen einer heterogenen Mischung aus Aktivkomponente oder Vorläuferverbindung hiervon mit einer Trägerkomponente oder Vorläuferverbindung hiervon erhalten werden.

Die Katalysatoren werden vorzugsweise als Formkörper eingesetzt. Beispiele umfassen Stränge, Rippstränge, andere Extrudatformen, Tabletten, Ringe, Kugeln und Splitt.

Bei der erfindungsgemäßen Hydrierung, bei der neben MSA andere, vorstehend definierte C₄-Dicarbonsäuren oder deren Derivate als Edukt eingesetzt werden können, werden beide Katalysatoren (erste und zweite Hydrierstufe) in reduzierter, aktivierter Form verwendet. Die Aktivierung erfolgt mit reduzierenden Gasen, vorzugsweise Wasserstoff oder Wasserstoff/Inertgas-Gemischen, entweder vor oder nach dem Einbau in den Reaktor, in dem das erfindungsgemäße Verfahren durchgeführt wird. Wurde der Katalysator in oxidischer Form in den Reaktor eingebaut, so kann die Aktivierung sowohl vor dem Anfahren der Anlage mit der erfindungsgemäßen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/Inertgas-Gemischen bei erhöhten Temperaturen, vorzugsweise zwischen 100 und 300°C. Bei der sogenannten in-situ-Aktivierung erfolgt die Aktivierung beim Hochfahren der Anlage durch Kontakt mit Wasserstoff bei erhöhter Temperatur.

Die BET-Oberfläche beider Kupferkatalysatoren beträgt im oxidischen Zustand 10 bis 400 m²/g, vorzugsweise 15 bis 200 m²/g, insbesondere 20 bis 150 m²/g. Die Kupferoberfläche (N₂O-Zersetzung) der reduzierten Katalysatoren beträgt im Einbauzustand > 0,2 m²/g, vorzugsweise > 1 m²/g, insbesondere > 2 m²/g.

Gemäß einer Variante der Erfindung werden Katalysatoren für beide Hydrierstufen verwendet, die eine definierte Porosität aufweisen. Diese Katalysatoren zeigen als Formkörper ein Porenvolumen von ≥ 0,01 ml/g für Porendurchmesser > 50 nm, vorzugsweise ≥ 0,025 ml/g für Porendurchmesser > 100 nm und insbesondere ≥ 0,05 ml/g für Porendurchmesser > 200 nm. Weiterhin liegt das Verhältnis von Makroporen mit einem Durchmesser > 50 nm zum Gesamtporenvolumen für Poren mit einem Durchmesser > 4 nm bei Werten > 10 %, bevorzugt > 20 %, insbesondere > 30 %. Oftmals lassen sich durch Verwendung dieser Katalysatoren hohe THF-Ausbeuten und - Selektivitäten erreichen. Die erwähnten Porositäten wurden durch Quecksilber-Intrusion nach DIN 66133 bestimmt. Es wurden die Daten im Porendurchmesserbereich von 4 nm bis 300 µm ausgewertet.

Die erfindungsgemäß verwendeten Katalysatoren für beide Hydrierstufen besitzen im allgemeinen eine ausreichende Standzeit. Für den Fall, dass die Aktivität und/oder Selektivität des Katalysators dennoch im Laufe ihrer Betriebszeit sinken sollte, kann dieser durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, den Katalysator mit einem geeigneten Lösungsmittel, beispielsweise Ethanol, THF oder GBL, zu waschen und anschließend in einem Gasstrom zu trocknen.

Das erfindungsgemäße Verfahren zeichnet sich dadurch aus, dass zu hydrierende Edukte unterschiedlicher Reinheit in die Hydrierreaktion eingesetzt werden können. Selbstverständlich kann ein Edukt hoher Reinheit, insbesondere MSA, in die Hydrierreaktion eingesetzt werden. Der erfindungsgemäß verwendete Katalysator sowie die sonstigen erfindungsgemäß gewählten Reaktionsbedingungen ermöglichen aber auch den Einsatz von Edukten, insbesondere MSA, das mit den üblichen, bei der Oxidation von Benzol, Butenen oder n-Butan anfallenden Verbindungen sowie eventuell weiteren Komponenten verunreinigt ist. Somit kann das erfindungsgemäße Hydrierverfahren in einer weiteren Ausführungsform eine vorgeschaltete Stufe umfassen, die das Herstellen des zu hydrierenden Edukts durch partielle Oxidation eines geeigneten Kohlenwasserstoffs sowie das Abtrennen des zu hydrierenden Edukts aus dem damit erhaltenen Produktstrom umfasst.

Insbesondere ist dieses zu hydrierende Edukt MSA. Dabei wird bevorzugt MSA eingesetzt, welches aus der Partialoxidation von Kohlenwasserstoffen stammt. Geeignete Kohlenwasserstoffströme sind Benzol, C₄-Olefine (z.B. n-Butene, C₄-Raffinatströme) oder n-Butan. Besonders bevorzugt eingesetzt wird n-Butan, da es einen preiswerten, wirtschaftlichen Einsatzstoff darstellt. Verfahren zur Partialoxidation von n-Butan sind beispielsweise in Ullmann's Encyclopedia of Industrial Chemistry, 6^{th} Edition, Electronic Release, Maleic and Fumaric Acids - Maleic Anhydride beschrieben.

Der so erhaltene Reaktionsaustrag wird dann in einem geeigneten organischen Lösungsmittel oder -Gemisch aufgenommen, das bei Atmosphärendruck einen um mindestens 30°C höheren Siedepunkt als MSA hat.

Dieses Lösungsmittel (Absorptionsmittel) wird auf eine Temperatur im Bereich zwischen 20 und 160°C, bevorzugt zwischen 30 und 80°C, gebracht. Der Maleinsäureanhydrid enthaltende Gasstrom aus der Partialoxidation kann in vielfältiger Weise mit dem Lösungsmittel in Kontakt gebracht werden: (i) Einleiten des Gasstroms in das Lösungsmittel (z.B. über Gaseinleitungsdüsen oder Begasungsringe), (ii) Einsprühen des Lösungsmittels in den Gasstrom und (iii) Gegenstromkontakt zwischen dem nach oben strömenden Gasstrom und dem nach unten strömenden Lösungsmittel in einer Boden-oder Packungskolonne. In allen drei Varianten können die dem Fachmann bekannten Apparate zur Gasabsorption eingesetzt werden. Bei der Wahl des einzusetzenden Lösungsmittels ist darauf zu achten, dass dies nicht mit dem Edukt, beispielsweise dem vorzugsweise eingesetzten MSA, reagiert. Geeignete Lösungsmittel sind: Trikresylhosphat, Dibutylmaleat, Butylmaleat, hochmolekulare Wachse, aromatische Kohlenwasserstoffe mit einem Molekulargewicht zwischen 150 und 400 und einem Siedepunkt oberhalb 140°C, wie beispielsweise Dibenzylbenzol; Dialkylphthalate mit C₁-C₈-Alkylgruppen, beispielsweise Dimethylphthalat, Diethylphthalat, Dibutylphthalat, Di-n-Propyl-und Di-iso-Propyl-phthalat; Di-C₁-C₄-Alkylester anderer aromatischer und alihatischer Dicarbonsäuren, beispielsweise Dimethyl-2,3-Naphthalin-Dicarbonsäure, Dimethyl-1,4-Cyclohexan-Dicarbonsäure, Alkylphthalate mit C₁-C₈-Alkylgruppen, beispielsweise Methylphthalat, Ethylphthalat, Butylphthalat, n-Propyl-und iso-Propyl-phthalat; C₁-C₄-Alkylester anderer aromatischer und aliphatischer Dicarbonsäuren, beispielsweise Methyl-2,3-Naphthalin-Dicarbonsäure, Methyl-1,4-Cyclohexan-DicarbonsäureMethylester langkettiger Fettsäuren mit beispielsweise 14 bis 30 Kohlenstoffatomen, hochsiedende Ether, beispielsweise Dimethylether von Polyethylenglykol, beispielsweise Tetraethylenglykoldimethylether.

Der Einsatz von Phthalaten ist bevorzugt.

Die nach der Behandlung mit dem Absorptionsmittel resultierende Lösung hat generell einen MSA-Gehalt von etwa 5 bis 400 Gramm pro Liter.

Der nach der Behandlung mit dem Absorptionsmittel verbleibende Abgasstrom enthält hauptsächlich die Nebenprodukte der vorangegangenen Partialoxidation, wie Wasser, Kohlenmonoxid, Kohlendioxid, nicht umgesetzte Butane, Essig- und Acrylsäure. Der Abgasstrom ist praktisch frei von MSA.

Anschließend wird das gelöste MSA aus dem Absorptionsmittel ausgetrieben. Dies erfolgt mit Wasserstoff bei oder maximal 10% oberhalb des Druckes der anschließenden Hydrierung oder alternativ im Vakuum mit anschließender Kondensation von verbleibendem MSA. In der Strippkolonne wird ein Temperaturprofil beobachtet, das sich aus den Siedepunkten von MSA am Kopf und dem nahezu MSA-freien Absorptionsmittel am Sumpf der Kolonne bei dem jeweiligen Kolonnendruck und der eingestellten Verdünnung mit Trägergas (im ersten Fall mit Wasserstoff) ergibt.

Um Verluste an Lösungsmittel zu verhindern, können sich oberhalb der Zufuhr des Roh-MSA-Stromes Rektifiziereinbauten befinden. Das vom Sumpf abgezogene, nahezu MSA-freie Absorptionsmittel wird wieder der Absorptionszone zugeführt. Das H₂/MSA-Verhältnis liegt bei etwa 20 bis 400. Im anderen Fall wird das kondensierte MSA in einen Verdampfer gepumpt und dort in den Kreisgasstrom verdampft.

Der MSA-Wasserstoff-Strom enthält noch Nebenprodukte, die bei der partiellen Oxidation von n-Butan, Butenen oder Benzol mit Sauerstoff enthaltenden Gasen entstehen, sowie nicht abgetrenntes Absorptionsmittel. Hierbei handelt es sich vor allem um Essigsäure und Acrylsäure als Nebenprodukte, Wasser, Maleinsäure sowie die vorzugsweise als Absorptionsmittel verwendeten Dialkylphthalate. Das MSA enthält Essigsäure in Mengen von 0,01 bis 1 Gew.%, vorzugsweise 0, 1 bis 0,8 Gew.-% und Acrylsäure in Mengen von 0,01 bis 1 Gew.-%, vorzugsweise 0,1 bis 0,8 Gew.-%, bezogen auf MSA. In der Hydrierstufe werden Essigsäure und Acrylsäure ganz oder teilweise zu Ethanol bzw. Propanol hydriert. Der Maleinsäure-Gehalt beträgt 0,01 bis 1 Gew.-%, insbesondere 0,05 bis 0,3 Gew.%, bezogen auf MSA.

Werden Dialkylphthalate als Absorptionsmittel eingesetzt, hängt deren Gehalt im MSA stark vom richtigen Betrieb der Strippkolonne, insbesondere vom Verstärkungsteil ab. Phthalatgehalte von bis 1,0 Gew.-% , insbesondere bis 0,5 Gew.-% sollten bei geeigneter Betriebsweise nicht überschritten werden, da sonst der Verbrauch an Absorptionsmittel zu hoch wird.

Der so erhaltene Wasserstoff/Maleinsäureanhydrid-Strom wird nun der ersten Hydrierzone zugeführt und hydriert. Die Katalysatoraktivitäten und -standzeiten sind dabei verglichen mit dem Einsatz von stark, beispielsweise durch Destillation, vorgereinigtem MSA praktisch unverändert. Das erfindungsgemäße Verfahren erlaubt BDO-Ausbeuten, die bei Werten von etwa 90% liegen. Es wird dabei auch eine hohe Produktselektivität erzielt.

Das erfindungsgemäße Verfahren wird nun in den nachfolgenden Beispielen näher erläutert.

### Beispiel 1:

### Herstellung eines erfindungsgemäßen Sprühpulvers

In einem beheizbaren und mit Rührwerk ausgestatteten Fälltopf werden 1,5 I Wasser vorgelegt und auf 80°C erwärmt. In dieses Fällgefäß werden im Verlauf einer Stunde eine Metallsalzlösung bestehend aus 877g Cu(NO₃)₂*2,5H₂O und 1472g Al(NO₃)₃*9H₂O in 2000 ml Wasser und gleichzeitig eine 20 Gew.-% Sodalösung unter Rühren zudosiert. Die Sodadosierung wird so gewählt, dass sich im Fällgefäß ein pH-Wert von 6 einstellt. Nach vollständiger Zugabe der Metallsalzlösung wird weiter Sodalösung zudosiert, bis im Fällgefäß ein pH-Wert von 8 erreicht ist und weitere 15 min bei diesem pH-Wert gerührt. Der Gesamtverbrauch an Sodalösung liegt bei 5,5 kg. Die gebildete Suspension wird abfiltriert und mit Wasser gewaschen, bis das ablaufende Waschwasser kein Nitrat (< 25 ppm) mehr enthält. Der Filterkuchen wird mit Wasser aufgeschlämmt. Die entstehende Maische wird bei 120 bis 135°C versprüht.

### Beispiel 2:

### Herstellung kleiner Formkörper

a) Tabletten (1,5 x 2 mm)
   Formkörpervolumen : 3,5 mm³
   Das Sprühpulver aus Beispiel 1 wird anschließend bei 600°C calciniert. Der so hergestellte Katalysator enthält 61 Gew.-% CuO und 39 Gew.-% Al₂O₃.Dieser wird intensiv mit 3 % Graphit vermischt und zu Tabletten der Größe 1,5 x 2 mm verpresst. Diese Tabletten enthalten 59 Gew.-% CuO, 38 Gew.-% Al₂O₃ und 3 Gew.-% Kohlenstoff.
b) Extrudate
   Formkörpervolumen: 3,5 - 5,3 mm³
   Böhmit wird mit Ameisensäure angeätzt, mit Sprühpulver aus Beispiel 1 vermischt und nach Zugabe von Wasser in einem Extruder zu Strängen der Länge 3 mm mit einem Durchmesser von 1,5 mm extrudiert. Die Stränge werden anschließend getrocknet und bei 600 °C calciniert. Die Extrudate enthalten 50 Gew.-% CuO und 50 Gew.-% Al₂O₃.

### Beispiel 3:

### Herstellung des Katalysators für die zweite Hydrierstufe:

### Herstellung des Trägers

Zu 649 g einer gut gerührten wässrigen Lösung von Zinknitrat mit einem Zink-Gehalt von 14,5 Gew.-% werden 450 g Al(NO₃)₃ * 9 H₂O zugegeben und das Gemisch mit Wasser auf ein Volumen von 1,25 I gebracht, um das Aluminiumsalz in Lösung zu bringen (Lösung A). In einem separaten Gefäß werden 474 g wasserfreies Soda in Wasser gelöst und die Lösung auf 2 I mit Wasser aufgefüllt. (Lösung B).

Lösung A und Lösung B werden auf 50°C erhitzt und über getrennte Leitungen in ein Fällgefäß, das eine auf 50°C erhitzte, gut gerührte Lösung von 20 g NaHCO₃ in 350 ml Wasser enthielt, geleitet. Hierbei wird durch entsprechende Einstellung der Zufuhrgeschwindigkeiten der Lösungen A und B der pH-Wert innerhalb von ca. 3 Minuten auf 6,8 gebracht. Unter Konstanthaltung des pH-Wertes bei 6,8 und der Temperatur bei 50°C wird die gesamte Lösung A mit Soda zur Reaktion gebracht. Die so gebildete Suspension wird anschließend 3 Stunden lang nachgerührt, wobei der pH-Wert durch gelegentliche Zugabe von verdünnter Salpetersäure bei 6,8 gehalten wird. Die Suspension wird filtriert und mit destilliertem Wasser so lange gewaschen, bis der Nitratgehalt des Waschwassers < 10 ppm beträgt. Der Filterkuchen wird 16 h lang bei 120°C getrocknet und anschließend 1 h lang bei 425°C calciniert.

### Herstellung des Katalysators

Ein Gemisch aus 432 g einer salpetersauren Kupfernitratlösung mit einem Kupfergehalt von 15,5 Gew.-% und 95 g einer salpetersauren Zinknitratlösung mit einem Zink-Gehalt von 14,5 Gew.-% wird mit Wasser auf 500 ml verdünnt und auf 70°C erwärmt. Unter Rühren werden 25,1 g des oben beschriebenen pulverförmigen calcinierten Trägers während ca. 5 Minuten langsam zugegeben und die so erhaltene milchige Suspension 15 Minuten lang gerührt (Suspension C).

In einem separaten Gefäß werden 474 g wasserfreies Soda in Wasser gelöst und die Lösung auf 2 1 mit Wasser aufgefüllt und auf 70°C erhitzt (Lösung D). Suspension C und Lösung D werden über getrennte Leitungen in ein Fällgefäß, das mit einem Rührer versehen ist und 350 ml auf 70°C erhitztes Wasser enthält, geleitet. Hierbei wird durch entsprechende Einstellung der Zufuhrgeschwindigkeiten der Suspension C und Lösung D der pH-Wert auf 7,4 gebracht.

Unter Konstanthaltung des pH-Wertes bei 7,4 und der Temperatur bei 70°C wird die gesamte Suspension C mit Soda zur Reaktion gebracht. Die so gebildete Suspension wird anschließend 2 Stunden lang nachgerührt, wobei der pH-Wert durch gelegentliche Zugabe von verdünnter Salpetersäure bzw. Sodalösung D bei 7,4 gehalten wird. Die Suspension wird filtriert und mit destilliertem Wasser so lange gewaschen, bis der Nitratgehalt des Waschwassers < 10 ppm beträgt.

Der Filterkuchen wird 16 h lang bei 120°C getrocknet und anschließend 1 h lang bei 430°C calciniert. Das so erhaltene braunschwarze Katalysatorpulver wird mit 1,5 Gew.-% Graphit und 5 Gew.-% Kupferpulver (Typ FFL Nr. 10914 der Norddeutschen Affinerie mit einer BET-Oberfläche von 0,23 m²/g und einer Partikelgrößenverteilung, bei der 92% der Partikel im Größenbereich 10 bis 100 µm liegen) gemischt und zu Tabletten von 3 mm Durchmesser und 3 mm Höhe verpresst. Die Tabletten werden schließlich 1 h lang bei 330°C calciniert. Der so hergestellte Katalysator hat die chemische Zusammensetzung 66 % CuO / 24 % ZnO / 5 % Al₂O₃ / 5 % Cu.

### Beispiel 4

### Durchführung der MSA-Hydrierung (1. Hydrierstufe) mit dem erfindungsgemäßen Katalysator aus Beispiel 2a)

### a) Katalysator-Aktivierung

Vor dem Reaktionsbeginn wird der Katalysator in der drucklosen Hydrierapparatur einer Wasserstoffbehandlung unterzogen. Hierzu wird der Reaktor auf 200°C temperiert und der Katalysator die in Tabelle 1 angegebene Zeit mit dem jeweils angegebenen Gemisch aus Wasserstoff und Stickstoff bei Atmosphärendruck aktiviert.

**Tabelle 1**

| Zeit (Minuten) | Wasserstoff (NI/h) | Stickstoff (NI/h) |
|---|---|---|
| 600 | 50 | 800 |
| 840 | 50 | 400 |
| 15 | 200 | 0 |

Anschließend wird der 15 h bei 250°C mit 200 NI/h Wasserstoff umspült.

### b) Versuchsanlage

Die zur Hydrierung verwendete Druckapparatur besteht aus einem Verdampfer, einem Reaktor, einem Kühler, einer Wasserstoffzufuhr, einer Abgasleitung und einem Kompressor. Der Druck in der Apparatur wird konstant gehalten.

Das aufgeschmolzene MSA wird von oben durch ein Einsteckrohr auf den vorgeheizten (195°C) Verdampfer gepumpt und verdampft. Auf den Verdampfer gelangt von unten eine vorgeheizte Mischung aus frischem Wasserstoff und Kreisgas. Beispiel Wasserstoff und MSA gelangen in den mit Katalysator gefüllten temperierten Reaktor (Durchmesser 20 mm). Nach der Hydrierung verlässt das entstandene Gemisch aus GBL und THF zusammen mit Wasser, anderen Reaktionsprodukten und Wasserstoff den Reaktor und wird im Kühler niedergeschlagen. Ein Teil des Kreisgases wird ausgeschleust, bevor der Rest, mit Frischwasserstoff vermischt, wieder in den Verdampfer eintritt.

In dieser Versuchsanlage wird der Reaktor mit 1800 ml des Katalysators aus Beispiel 2a (Gesamtmasse 1575 g) gefüllt und entsprechend 4a) aktiviert.

Der kondensierte flüssige Reaktionsaustrag, das Abgas und das Kreisgas werden gaschromatographisch quantitativ analysiert.

Die Betriebsparameter und Versuchsergebnisse sind Tabelle 2 zu entnehmen.

**Tabelle 2**

| Experiment | Druck [bar] | Belastung [kgMSA/L Kath] | Temp. [°C] | THF [mol-%] | GBL [mol%] | Butanol [mol%] | BSA [mol.-%] |
|---|---|---|---|---|---|---|---|
| 1 | 5 | 0,2 | 249 | 20,2 | 76,4 | 0,8 | 0,7 |
| 2 | 5 | 0,2 | 251 | 40,5 | 56,0 | 1,8 | 0,4 |
| 3 | 5 | 0,2 | 253 | 62,2 | 33,9 | 2,6 | 0 |
| 4 | 10 | 0,3 | 251 | 15,1 | 82,1 | 0,6 | 0,6 |
| 5 | 10 | 0,3 | 253 | 55,5 | 41,5 | 2,0 | 0,2 |
| 6 | 10 | 0,3 | 254 | 82,4 | 12,5 | 3,5 | 0,1 |
| 7 | 10 | 0,3 | 257 | 93,5 | 0,2 | 5,2 | 0 |
| 8 | 25 | 0,4 | 249 | 36,5 | 61,1 | 0,6 | 0,8 |
| 9 | 25 | 0,4 | 250 | 40,8 | 55,8 | 0,9 | 0,6 |
| 10 | 25 | 0,4 | 251 | 49,8 | 48,0 | 0,7 | 0,3 |
| 11 | 25 | 0,4 | 255 | 97,7 | 0 | 1,8 | 0 |

### Beispiel 5:

### Durchführung der MSA-Hydrierung (1. Hydrierstufe) mit dem erfindungsgemäßen Katalysator aus Beispiel 2b)

### a) Katalysator-Aktivierung

Vor dem Reaktionsbeginn wird der Katalysator in der drucklosen Hydrierapparatur einer Wasserstoffbehandlung unterzogen. Hierzu wird der Reaktor auf 200°C temperiert und der Katalysator die in Tabelle 3 angegebene Zeit mit dem jeweils angegebenen Gemisch aus Wasserstoff und Stickstoff bei Atmosphärendruck aktiviert.

**Tabelle 3**

| Zeit (Minuten) | Wasserstoff (Nl/h) | Stickstoff (Nl/h) |
|---|---|---|
| 600 | 50 | 800 |
| 840 | 50 | 400 |
| 15 | 200 | 0 |

Anschließend wird der 15 h bei 250°C mit 200 Nl/h Wasserstoff umspült.

### b) Versuchsanlage

Die zur Hydrierung verwendete Druckapparatur besteht aus einem Verdampfer, einem Reaktor, einem Kühler, einer Wasserstoffzufuhr, einer Abgasleitung und einem Kompressor. Der Druck in der Apparatur wird konstant gehalten.

Das aufgeschmolzene MSA wird von oben durch ein Einsteckrohr auf den vorgeheizten (195°C) Verdampfer gepumpt und verdampft. Auf den Verdampfer gelangt von unten eine vorgeheizte Mischung aus frischem Wasserstoff und Kreisgas. Beispiel Wasserstoff und MSA gelangen in den mit Katalysator gefüllten temperierten Reaktor (Durchmesser 34 mm). Nach der Hydrierung verlässt das entstandene Gemisch aus GBL und THF zusammen mit Wasser, anderen Reaktionsprodukten und Wasserstoff den Reaktor und wird im Kühler niedergeschlagen. Ein Teil des Kreisgases wird ausgeschleust, bevor der Rest, mit Frischwasserstoff vermischt, wieder in den Verdampfer eintritt.

In dieser Versuchsanlage wird der Reaktor mit 1200 ml des Katalysators aus Beispiel 2b gefüllt und entsprechend 5a) aktiviert.

Der kondensierte flüssige Reaktionsaustrag, das Abgas und das Kreisgas werden gaschromatographisch quantitativ analysiert.

Die Betriebsparameter und Versuchsergebnisse sind Tabelle 4 zu entnehmen.

**Tabelle 4**

| Experiment | Betriebsdauer [h] | Druck [bar] | Belastung [kgMSA/ LKath] | Temp. [°C] | THF [mol%] | GBL [mol%] | Butanol [mol%] | BSA [mol.-%] |
|---|---|---|---|---|---|---|---|---|
| 12 | | | | | | | | |
| 13 | 1226 | 9 | 0,2 | 245 | 53,6 | 43,9 | 1,1 | 0 |
| 14 | 3081 | 9 | 0,2 | 246 | 53,4 | 42,8 | 2,1 | 0 |
| 15 | 3972 | 9 | 0,2 | 247 | 54,7 | 40,4 | 2,6 | 0,2 |

### Beispiel 6

### Durchführung der 2. Hydrierstufe mit dem Katalysator aus Beispiel 3 (Feed: Produktaustrag Beispiel 4, Experiment 9)

### b) Katalysator-Aktivierung

Vor dem Reaktionsbeginn wird der Katalysator in der Hydrierapparatur einer Wasserstoffbehandlung unterzogen. Hierzu wird der Reaktor auf 180°C temperiert und der Katalysator die in Tabelle 1 angegebene Zeit mit dem jeweils angegebenen Gemisch aus Wasserstoff und Stickstoff bei Atmosphärendruck aktiviert.

**Tabelle 5**

| Zeit (Minuten) | Wasserstoff (Nl/h) | Stickstoff (Nl/h) |
|---|---|---|
| 120 | 10 | 550 |
| 30 | 25 | 400 |
| 15 | 60 | 100 |
| 180 | 60 | 0 |

### c) Hydrierapparatur:

Der Produktaustrag des Experiments 9, Beispiel 4 wird in einen vorgeheizten Verdampfer gepumpt und dort im Gegenstrom mit einer Mischung aus Wasserstoff und Kreisgas verdampft. Das gasförmige Gemisch aus Wasserstoff und Feed gelangt anschließend in den temperierten Reaktor. Der Reaktor enthält ein Gemisch aus Glasringen und Katalysator. Nach der Hydrierung verlassen die Reaktionsprodukte und Wasserstoff den Reaktor. Die Reaktionsprodukte werden niedergeschlagen. Ein Teil des Kreisgases wird ausgeschleust, bevor der Rest, mit Frischwasserstoff vermischt, wieder in den Verdampfer eintritt.

Der Reaktor der in Beispiel 1 c beschriebenen Hydrierapparatur wird mit 100 ml des nach Beispiel 3 hergestellten Katalysators und 100 ml Glasringen gefüllt. Die Aktivierung erfolgte wie in Beispiel 6b beschrieben. Als Edukt wird der Produktaustrag des Experiments 9, Beispiel 3 (55,8 FI-% GBL und 40,8 FI% THF, 0,9 FI% BuOH, 0,6 FI% BSA; bzw.: 45,5 Gew.-% GBL, 25,9 Gew.-% THF, 0,6 Gew.-% BSA, 0,5 Gew.-% Bu-OH, 0,4 Gew.-% Buttersäure und 26 Gew.-% Wasser bezogen auf die Gesamtmasse) eingesetzt. Die Reaktion wird bei unterschiedlichen Temperaturen und unterschiedlichen Drücken durchgeführt. In Tabelle 6 sind die Belastungen und die Ergebnisse der Hydrierung zusammengefasst.

**Tabelle 6**

| Experime | Druck [bar] | Belastung [kgMSA/ LKath] | Temp. [°C] | THF [mol-%] | GBL [mol%] | BDO [mol-%] | Butanol [mol-%] | BSA [mol.-%] |
|---|---|---|---|---|---|---|---|---|
| nt | 25 | 0,2 | 180 | 36,2 | 21,0 | 40,0 | 1,4 | 0 |
| | 25 | 0,3 | 180 | 40,8 | 33,7 | 23,0 | 1,8 | 0 |
| | 25 | 0,15 | 220 | 39,76 | 29,73 | 27,4 | 2,2 | 0 |
| | 25 | 0,3 | 220 | 41,5 | 37,8 | 18,0 | 2,0 | 0 |
| | 25 | 0,15 | 260 | 53,3 | 26,9 | 5,8 | 9,8 | 0 |
| | 25 | 0,3 | 260 | 47,0 | 38,2 | 7,8 | 4,9 | 0 |
| | 10 | 0,1 | 180 | 37,5 | 41,7 | 18,6 | 1,5 | 0 |
| | 10 | 0,1 | 220 | 43,5 | 47,4 | 5,9 | 3,4 | 0 |
| | 10 | 0,1 | 260 | 48,5 | 37,2 | 1,6 | 8,7 | 0 |

## Patentansprüche

1. Verfahren zur variablen Herstellung von Gemischen von gegebenenfalls alkylsubstituiertem BDO, GBL und THF durch zweistufige Hydrierung in der Gasphase von C₄-Dicarbonsäuren und/oder deren Derivaten, **dadurch gekennzeichnet, dass** man
a) in einem ersten Schritt in der Gasphase einen Gasstrom von C₄-Dicarbonsäuren und/oder deren Derivaten an einem Katalysator bei einem Druck von 2 bis 100 bar und einer Temperatur von 200°C bis 300°C in einem ersten Reaktor in Gegenwart eines Katalysator in Form von Katalysatorformkörpern mit einem Volumen kleiner als 20 mm³, die 5 bis 95 Gew.-% Cu-Oxid und 5 bis 95 Gew.-% eines Oxids mit sauren Zentren zu einem hauptsächlich aus gegebenenfalls alkylsubstituiertem GBL und THF haltigen Strom hydriert,
b) eventuell entstandenes Bernsteinsäureanhydrid durch Partialkondensation abtrennt,
c) die bei der Partialkondensation überwiegend in der Gasphase verbliebenen Produkte THF, Wasser und GBL unter gleichem oder um die Strömungsverluste im Hydrierkreislauf verringerten Druck und einer Temperatur von 150 bis 240°C in einem zweiten Reaktor an einem Katalysator der 5 bis 95 Gew.-% CuO und 5 bis 95 Gew.-% eines oder mehrerer Oxide ausgewählt aus der Gruppe ZnO, AL₂O₃, SiO₂, TiO₂, ZrO₂, CeO₂, MgO, CaO, SrO, BaO, La₂O₃ und Mn₂O₃ zu einem Gemisch aus BDO, GBL und THF enthaltenden Strom umsetzt.
d) den Wasserstoff von den Produkten abtrennt und in die Hydrierung zurückführt,
e) die Produkte THF, BDO, GBL und Wasser destillativ trennt, einen GBL-reichen Strom in den zweiten Reaktor gegebenenfalls zurückführt oder gegebenenfalls ausschleust und BDO, THF und GBL destillativ aufarbeitet,
und das Verhältnis der Produkte THF, GBL und BDO zueinander im Bereich von 10 bis 100 Gew.-% THF, 0 bis 90 Gew.-% GBL und 0 bis 90 Gew.-% BDO nur durch Variation der Temperaturen in beiden Hydrierzonen sowie gegebenenfalls des GBL-Rückführstroms eingestellt wird.

2. Verfahren nach Anspruch 1 **dadurch gekennzeichnet, dass** die Partialkondensation des BSA als Umlaufquenchkreis ausgeführt ist.

3. Verfahren nach den Ansprüchen 1 oder 2, **dadurch gekennzeichnet, dass** die Verdampfung des rückgeführten GBL oder GBL/Wasser-Gemisches in einem Gegenstromkontaktapparat mit dem GBL/THF-beladenen Kreisgaswasserstoff erfolgt.

4. Verfahren nach den Ansprüchen 1 bis 3 **dadurch gekennzeichnet, dass** die Partialkondensation des Bernsteinsäureanhydrides und die Verdampfung des GBL oder GBL/Wasser-Rückführstromes in einem Apparat kombiniert werden, wobei das Bernsteinsäureanhydrid zusammen mit Rest GBL, Wasser und hochsiedenden Nebenkomponenten als Sumpfaustrag ausgeschleust werden.

5. Verfahren nach einem der Ansprüche 1 bis 4 **dadurch gekennzeichnet, dass** das Verfahren kontinuierlich durchgeführt wird.

6. Verfahren nach einem der Ansprüche 1 bis 5, **dadurch gekennzeichnet, dass** die Eingangstemperatur im ersten Reaktor bei Werten von 200°C bis 300°C und 5 bis 15°C unterhalb der Hot-Spot-Temperatur liegt.

7. Verfahren nach einem der Ansprüche 1 bis 6, **dadurch gekennzeichnet, dass** die Temperaturerhöhung im zweiten Reaktor nicht mehr als 90°C beträgt.

8. Verfahren nach einem der Ansprüche 1 bis 7, **dadurch gekennzeichnet, dass** die Eingangstemperatur im zweiten Reaktor bei Werten zwischen 150°C und 270°C liegt.

9. Verfahren nach einem der Ansprüche 1 bis 8, **dadurch gekennzeichnet, dass** beide Hydrierstufen bei Drücken von 2 bis 100 bar durchgeführt werden.

10. Verfahren nach einem der Ansprüche 1 bis 9, **dadurch gekennzeichnet, dass** die Katalysatorbelastung der ersten Hydrierstufe im Bereich von 0,02 bis 2 kg Edukt /I Katalysator Stunde.

11. Verfahren nach einem der Ansprüche 1 bis 10, **dadurch gekennzeichnet, dass** die Katalysatorbelastung der zweiten Hydrierstufe im Bereich von 0,02 bis 2 kg Edukt /I Katalysator · Stunde.

12. Verfahren nach einem der Ansprüche 1 bis 11, **dadurch gekennzeichnet, dass** das Molverhältnis Wasserstoff/Edukt in der ersten Hydrierstufe bei Werten von 20 bis 650 liegt.

13. Verfahren nach einem der Ansprüche 1 bis 12, **dadurch gekennzeichnet, dass** das Molverhältnis Wasserstoff/GBL in der zweiten Hydrierstufe bei Werten von 20 bis 1000.

14. Verfahren nach einem der Ansprüche 1 bis 13, **dadurch gekennzeichnet, dass** in der ersten Hydrierstufe ein Festbettreaktor eingesetzt wird.

15. Verfahren nach einem der Ansprüche 1 bis 14, **dadurch gekennzeichnet, dass** in der zweiten Hydrierstufe ein Festbettreaktor eingesetzt wird.

16. Verfahren nach einem der Ansprüche 1 bis 15, **dadurch gekennzeichnet, dass** das Volumen des einzelnen Formkörpers in der ersten Hydrierstufe < 10 mm³ ist.

17. Verfahren nach einem der Ansprüche 1 bis 16, **dadurch gekennzeichnet, dass** der CuO < 80 Gew.-% mit sauren Zentren enthalten sind.

18. Verfahren nach einem der Ansprüche 1 bis 17, **dadurch gekennzeichnet, dass** das Oxid mit sauren Zentren Al₂O₃ ist.

19. Verfahren nach einem der Ansprüche 1 bis 18, **dadurch gekennzeichnet; dass** der Katalysator der ersten Hydrierstufe ein oder mehrere weitere Metalle oder eine Verbindung davon, aus der Gruppe bestehend aus den Elementen der Gruppen 1 bis 14 des Periodensystems der Elemente in dem Katalysator vorhanden sind.

20. Verfahren nach einem der Ansprüche 1 bis 19 **dadurch gekennzeichnet, dass** der Katalysatorformkörper als Strang vorliegt.

21. Verfahren nach einem der Ansprüche 1 bis 20, **dadurch gekennzeichnet, dass** der Katalysator der zweiten Hydrierstufe neben CuO Trägermaterialien ausgewählt aus der Gruppe ZnO/Al₂O₃-Gemische, delta-, theta-, alpha- und eta-Modifikationen des Al₂O₃, Mischungen, die a) Al₂O₃ und mindestens eine Komponente b) aus der Gruppe SiO₂, TiO₂, ZrO₂ einerseits und aus der Gruppe ZnO, MgO, CaO, SrO und BaO andererseits enthalten enthalten

22. Verfahren nach einem der Ansprüche 1 bis 4, **dadurch gekennzeichnet, dass** der Katalysator der zweiten Hydrierstufe Oxide ausgewählt aus ZnO, ZnO/Al₂O₃-Gemischen im Gewichtsverhältnis 100:1 bis 1:2 und Mischungen von SiO2 mit MgO, CaO und/oder ZnO im Gewichtsverhältnis 200:1 bis 1:1 enthalten.

23. Verfahren nach einem der Ansprüche 1 bis 20, **dadurch gekennzeichnet, dass** in einem zusätzlichen Schritt der Katalysator vor oder nach dem Einbau in den Reaktor und vor dem Einsatz in die Hydrierreaktion durch Reduktion aktiviert wird.

## Claims

1. A process for variably preparing mixtures of optionally alkyl-substituted BDO, GBL and THF by two-stage hydrogenation in the gas phase of C₄ dicarboxylic acids and/or derivatives thereof, which comprises
a) in a first step in the gas phase, hydrogenating a gas stream of C₄ dicarboxylic acids and/or derivatives thereof over a catalyst at a pressure of from 2 to 100 bar and a temperature of from 200°C to 300°C in a first reactor in the presence of a catalyst in the form of shaped catalyst bodies having a volume of less than 20 mm³, said catalyst comprising from 5 to 95% by weight of oxide of copper and from 5 to 95% by weight of an oxide having acidic sites, to give a stream mainly comprising optionally alkyl-substituted GBL and THF,
b) removing any succinic anhydride formed by partial condensation,
c) converting the products remaining predominantly in the gas phase in the partial condensation, THF, water and GBL, under the same pressure or under a pressure reduced by the pressure drops in the hydrogenation circuit and at a temperature of from 150 to 240°C, in a second reactor over a catalyst which comprises from 5 to 95% by weight of CuO and from 5 to 95% by weight of one or more oxides selected from the group of ZnO, Al₂O₃, SiO₂, TiO₂, ZrO₂, CeO₂, MgO, CaO, SrO, BaO, La₂O₃ and Mn₂O₃ to give a stream comprising a mixture of BDO, GBL and THF,
d) removing the hydrogen from the products and recycling it into the hydrogenation,
e) distillatively separating the products, THF, BDO, GBL and water, optionally recycling a GBL-rich stream into the second reactor or optionally discharging it, and working up BDO, THF and GBL distillatively,
and setting the ratio of the products, THF, GBL and BDO, relative to one another within the range from 10 to 100% by weight of THF, from 0 to 90% by weight of GBL and from 0 to 90% by weight of BDO only by varying the temperatures in the two hydrogenation zones and also any GBL recycle stream.

2. The process according to claim 1, wherein the partial condensation of the SA is designed as a circulation quench cycle.

3. The process according to claims 1 or 2, wherein the evaporation of the recycled GBL or GBL/water mixture is effected in a countercurrent apparatus with the GBL/THF-laden cycle gas hydrogen.

4. The process according to claims 1 to 3, wherein the partial condensation of the succinic anhydride and the evaporation of the GBL or GBL/water recycle stream are combined in one apparatus, and the succinic anhydride is discharged as the bottom effluent together with residual GBL, water and high-boiling secondary components.

5. The process according to any of claims 1 to 4, which is carried out continuously.

6. The process according to any one of claims 1 to 5, wherein the inlet temperature in the first reactor is at values of from 200°C to 300°C and from 5 to 15°C below the hotspot temperature.

7. The process according to any of claims 1 to 6, wherein the temperature increase in the second reactor is not more than 90°C.

8. The process according to any of claims 1 to 7, wherein the inlet temperature in the second reactor is at values between 150°C and 270°C.

9. The process according to any of claims 1 to 8, wherein both hydrogenation stages are carried out at pressures of from 2 to 100 bar.

10. The process according to any of claims 1 to 9, wherein the catalyst hourly space velocity of the first hydrogenation stage is in the range from 0.02 to 2 kg of reactant/1 of catalyst · hour.

11. The process according to any of claims 1 to 10, wherein the catalyst hourly space velocity of the second hydrogenation stage is in the range from 0.02 to 2 kg of reactant/l of catalyst · hour.

12. The process according to any of claims 1 to 11, wherein the molar hydrogen/reactant ratio in the first hydrogenation stage is at values of from 20 to 650.

13. The process according to any of claims 1 to 12, wherein the molar hydrogen/GBL ratio in the second hydrogenation stage is at values of from 20 to 1000.

14. The process according to any of claims 1 to 13, wherein the reactor used in the first hydrogenation stage is a fixed bed reactor.

15. The process according to any of claims 1 to 14, wherein the reactor used in the second hydrogenation stage is a fixed bed reactor.

16. The process according to any of claims 1 to 15, wherein the volume of the individual shaped body in the first hydrogenation stage is < 10 mm³.

17. The process according to any of claims 1 to 16, wherein the CuO < 80% by weight having acidic sites are present.

18. The process according to any of claims 1 to 17, wherein the oxide having acidic sites is Al₂O₃.

19. The process according to any of claims 1 to 18, wherein one or more further metals or a compound thereof, from the group consisting of the elements of groups 1 to 14 of the Periodic Table of the Elements are present in the catalyst of the first hydrogenation stage.

20. The process according to any of claims 1 to 19, wherein the shaped catalyst body is in the form of an extrudate.

21. The process according to any of claims 1 to 20, wherein the catalyst of the second hydrogenation stage comprises, in addiction to CuO, support materials selected from the group of ZnO/Al₂O₃ mixtures, delta-, theta-, alpha- and eta-modifications of Al₂O₃, mixtures which comprise a) Al₂O₃ and at least one component b) from the group of SiO₂, TiO₂, ZrO₂ on the one hand and from the group of ZnO, MgO, CaO, SrO and BaO on the other.

22. The process according to any of claims 1 to 4, wherein the catalyst of the second hydrogenation stage comprises oxides selected from ZnO, ZnO/Al₂O₃ mixtures in a weight ratio of from 100:1 to 1:2 and mixtures of SiO₂ with MgO, CaO and/or ZnO in a weight ratio of from 200:1 to 1:1.

23. The process according to any of claims 1 to 20, wherein, in an additional step, the catalyst is activated by reduction before or after installation into the reactor and before use in the hydrogenation reaction.

## Revendications

1. Procédé pour la préparation variable de mélanges de BDO (butanediol), de GBL (butyrolactone) et de THF (tétrahydrofuranne), le cas échéant substitués par alkyle, par hydrogénation en deux étapes en phase gazeuse d'acides C₄-dicarboxyliques et/ou leurs dérivés, **caractérisé en ce que**
a) dans une première étape, dans la phase gazeuse, on hydrogène un flux gazeux d'acides C₄-dicarboxyliques et/ou leurs dérivés sur un catalyseur à une pression de 2 à 100 bars et une température de 200°C à 300°C dans un premier réacteur en présence d'un catalyseur sous forme de corps façonnés catalytiques présentant un volume inférieur à 20 mm³, qui contiennent 5 à 95% en poids d'oxyde de cuivre et 5 à 95% en poids d'un oxydes avec des centres acides, en un flux qui contient principalement du GBL et du THF, le cas échéant substitués par alkyle,
b) on sépare de l'anhydride de l'acide succinique éventuellement formé par condensation partielle,
c) on transforme les produits THF, eau et GBL restant principalement dans la phase gazeuse lors de la condensation partielle à la même pression ou à la pression réduite des pertes d'écoulement dans le circuit d'hydrogénation et à une température de 150 à 240°C dans un deuxième réacteur sur un catalyseur qui contient 5 à 95% en poids de CuO et 5 à 95% en poids d'un ou de plusieurs oxydes choisis dans le groupe formé par ZnO, Al₂O₃, SiO₂, TiO₂, ZrO₂, CeO₂, MgO, CaO, SrO, BaO, La₂O₃ et Mn₂O₃, en un flux contenant un mélange de BDO, de GBL et de THF,
d) on sépare l'hydrogène des produits et on le recycle dans l'hydrogénation,
e) on sépare les produits THF, BDO, GBL et eau par distillation, on recycle le cas échéant un flux riche en GBL dans le deuxième réacteur ou on le soutire le cas échéant et on traite le BDO, le THF et le GBL par distillation,
et on règle le rapport des produits THF, GBL et BDO les uns par rapport aux autres dans la plage de 10 à 100% en poids de THF, 0 à 90% en poids de GBL et 0 à 90% en poids de BDO uniquement par variation des températures dans les deux zones d'hydrogénation ainsi que le cas échéant du flux de recyclage de GBL.

2. Procédé selon la revendication 1, **caractérisé en ce que** la condensation partielle du BSA (anhydride de l'acide succinique) est réalisée sous forme de circuit de désactivation en circulation.

3. Procédé selon les revendications 1 ou 2, **caractérisé en ce que** l'évaporation du GBL ou du mélange GBL/eau recyclé a lieu dans un appareil de contact à contre-courant avec l'hydrogène gazeux en circulation chargé de GBL/THF.

4. Procédé selon les revendications 1 à 3, **caractérisé en ce que** la condensation partielle de l'anhydride de l'acide succinique et l'évaporation du flux de recyclage de GBL ou de GBL/eau sont combinés dans un appareil, l'anhydride de l'acide succinique étant soutiré ensemble avec le GBL résiduel, l'eau et les composants secondaires à point d'ébullition élevé comme produit évacué du fond.

5. Procédé selon l'une quelconque des revendications 1 à 4, **caractérisé en ce que** le procédé est réalisé en continu.

6. Procédé selon l'une quelconque des revendications 1 à 5, **caractérisé en ce que** la température à l'entrée dans le premier réacteur se situe à des valeurs de 200°C à 300°C et 5 à 15°C sous la température de point chaud.

7. Procédé selon l'une quelconque des revendications 1 à 6, **caractérisé en ce que** l'augmentation de température dans le deuxième réacteur n'est pas supérieure à 90°C.

8. Procédé selon l'une quelconque des revendications 1 à 7, **caractérisé en ce que** la température à l'entrée du deuxième réacteur se situe à des valeurs entre 150°C et 270°C.

9. Procédé selon l'une quelconque des revendications 1 à 8, **caractérisé en ce que** les deux étapes d'hydrogénation sont réalisées à des pressions de 2 à 100 bars.

10. Procédé selon l'une quelconque des revendications 1 à 9, **caractérisé en ce que** la charge du catalyseur de la première étape d'hydrogénation se situe dans la plage de 0,02 à 2 kg de produit de départ /1 de catalyseur*heure.

11. Procédé selon l'une quelconque des revendications 1 à 10, **caractérisé en ce que** la charge du catalyseur de la deuxième étape d'hydrogénation se situe dans la plage de 0,02 à 2 kg de produit de départ /1 de catalyseur*heure.

12. Procédé selon l'une quelconque des revendications 1 à 11, **caractérisé en ce que** le rapport molaire hydrogène/produit de départ dans la première étape d'hydrogénation se situe à des valeurs de 20 à 650.

13. Procédé selon l'une quelconque des revendications 1 à 12, **caractérisé en ce que** le rapport molaire hydrogène/GBL dans la deuxième étape d'hydrogénation se situe à des valeurs de 20 à 1000.

14. Procédé selon l'une quelconque des revendications 1 à 13, **caractérisé en ce qu'**on utilise, dans la première étape d'hydrogénation, un réacteur à lit fixe.

15. Procédé selon l'une quelconque des revendications 1 à 14, **caractérisé en ce qu'**on utilise, dans la deuxième étape d'hydrogénation, un réacteur à lit fixe.

16. Procédé selon l'une quelconque des revendications 1 à 15, **caractérisé en ce que** le volume d'un corps façonné individuel dans la première étape d'hydrogénation est < 10 mm³.

17. Procédé selon l'une quelconque des revendications 1 à 16, **caractérisé en ce que** le CuO < 80% en poids avec des centres acides sont presents.

18. Procédé selon l'une quelconque des revendications 1 à 17, **caractérisé en ce que** l'oxyde contenant les centres acides est Al₃O₃.

19. Procédé selon l'une quelconque des revendications 1 à 18, **caractérisé en ce qu'**un ou plusieurs autres métaux ou un composé de ceux-ci, du groupe constitué par les éléments des groupes 1 à 14 du système périodique des éléments sont présents dans le catalyseur de la première étape d'hydrogénation.

20. Procédé selon l'une quelconque des revendications 1 à 19, **caractérisé en ce que** le corps façonné catalytique se trouve sous forme de brin.

21. Procédé selon l'une quelconque des revendications 1 à 20, **caractérisé en ce que** le catalyseur de la deuxième étape d'hydrogénation contient, outre le CuO, des matériaux support choisis dans le groupe des mélanges ZnO/Al₂O₃, des modifications delta, thêta, alpha et êta de l'Al₂O₃, des mélanges qui contiennent a) Al₂O₃ et au moins un composant b) du groupe SiO₂, TiO₂, ZrO₂ d'une part et du groupe ZnO, MgO, CaO, SrO et BaO d'autre part.

22. Procédé selon l'une quelconque des revendications 1 à 4, **caractérisé en ce que** le catalyseur de la deuxième étape d'hydrogénation contient des oxydes choisis parmi ZnO, des mélanges de ZnO/Al₂O₃ dans un rapport pondéral de 100:1 à 1:2 et des mélanges de SiO₂ avec MgO, CaO et/ou ZnO dans un rapport pondéral 200:1 à 1:1.

23. Procédé selon l'une quelconque des revendications 1 à 20, **caractérisé en ce que** dans une étape supplémentaire, le catalyseur est activé par réduction avant ou après l'incorporation dans le réacteur et avant l'utilisation dans la réaction d'hydrogénation.
